# EUROPEAN PATENT SPECIFICATION

(11) **EP 0 375 736 B1**
(45) Date of publication and mention of the grant of the patent: **13.05.1998**
(21) Application number: 88908472.9
(22) Date of filing: 05.08.1988
(51) Int. Cl.: A61K 47/48, C12Q 1/68, C07K 14/47

(54) **NUCLEIC ACID FRAGMENTS ENCODING MANNOSE BINDING FRAGMENTS OF HUMAN MANNOSE BINDING PROTEIN**
NUKLEINSAÜREFRAGMENTE, DIE MANNOSE BINDENDE FRAGMENTE VOM MENSCHLICHEN MANNOSEBINDENDEN PROTEIN KODIEREN
FRAGMENTS D'ACIDE NUCLEIQUE CODANT DES FRAGMENTS DU PROTEINE HUMAINE LIANT LE MANNOSE

(30) Priority: 20.08.1987 US 87628
(43) Date of publication of application: 04.07.1990
(73) Proprietor: CHILDREN'S MEDICAL CENTER CORPORATION, Boston Massachusetts 02115 (US)
(72) Inventor: EZEKOWITZ, Raymond, Alan, Brian, Boston, MA 02116 (US)
(74) Representative: Crisp, David Norman
(86) International application number: US8802591
(87) International publication number: WO8901519

(56) References cited:
- WO-A-83/03971
- Chemical Abstracts, volume 105, no. 21, 24 November 1986, (Columbus, Ohio, US), J.A. Summerfield et al.: "Mannose-binding proteins in human serum: identification of mannose-specific immunoglobulins and a calcium-dependent lectin, of broader carbo-hydrate specificity, secreted by hepatocytes", see page 573, abstract 189211v
- The Journal of Biological Chemistry, volume 261, no. 15, 25 May 1986, The American Society of Biological Chemists, Inc., (Baltimore, US), K. Drickamer et al.: "Mannose-binding proteins isolated from rat liver contain carbohydrate-re-cognition domains linked to collagenous tails", pages 6878-6887
- The Journal of Biological Chemistry, volume 262, no. 6, 25 February 1987, The American Society of Biological Chemists, Inc., (Baltimore, US), K. Drickamer et al.: "Exon stucture of a mannose-binding protein gene reflects its evolutionary relationship to the asialoglycoprotein receptor and nonfibrillar collagens", pages 2582-2589
- The Journal of Biological Chemistry, volume 262, no. 29, 15 October 1987, The American Society for Biochemistry and Molecular Biology, (Baltimore, US), H.P. Haagsman et al.: "The major lung surfactant protein, SP 28-36, is a calcium-dependent, carbohydrate binding protein", pages 13877-13880
- Nature, volume 317, 26 September 1985, (London, GB), R. Tyler White et al.: "Isolation and characterization of the human pulmonary surfactant apoprotein gene", pages 361-363
- Chemical Abstracts, volume 109, no. 15, 10 October 1988, (columbus, Ohio, US), R. Ezekowitz et al.: "A human mannose-binding protein is an acute-phase reactant that shares sequence homology with other vertebrate lectins", see page 268, abstract 124567d
- Chemical Abstracts, volume 109, no. 11, 12 September 1988, (Columbus, Ohio, US), R. Ezekowitz et al.: "The role of human mannose-lectin-like molecules in host defense", see page 517, abstract 90799p

## Description

### Background of the Invention

This invention relates to nucleic acid fragments encoding for protein fragments able to bind mannose.

Mannose-binding proteins (MBPs) have been isolated from rabbit, rat, and human liver. Taylor et al. Clinical Science 70:539, 1986. MBPs have also been found in serum, and may play a role in the disposal of pathogenic organisms. Id.

Summerfield et al. Bioc. Biop. Acta 883:197, 1986 describe two types of MBP's in human serum. These were detected using antibodies raised against a 30 kDa subunit of one MBP. The authors suggest that MBPs may bind noxious glycoproteins in the circulation prior to the removal of these glycoproteins; and that yeasts and bacteria contain glycoproteins which are bound by MBPs.

Stahl et al. Biol. Cell 51:215, 1984 describe a mannose receptor, which is distinct from MBPs. These two proteins appear to be structurally related since antibodies to one protein may react with the other protein.

Wild et al. Biochem. J. 210:167, 1983 describe the isolation of MBP from human and rat liver. The human MBP has a molecular weight greater than one million and consists of 28 kDa and 30.5 kDa subunits.

Drickamer et al. J. Biol. Chem. 261:6878, 1986 describes the isolation of MBPs from rat liver, and the cloning of cDNAs encoding these proteins. Each MBP has a cysteine rich region, a collagen-like domain and a carbohydrate binding domain.

### Summary of Invention

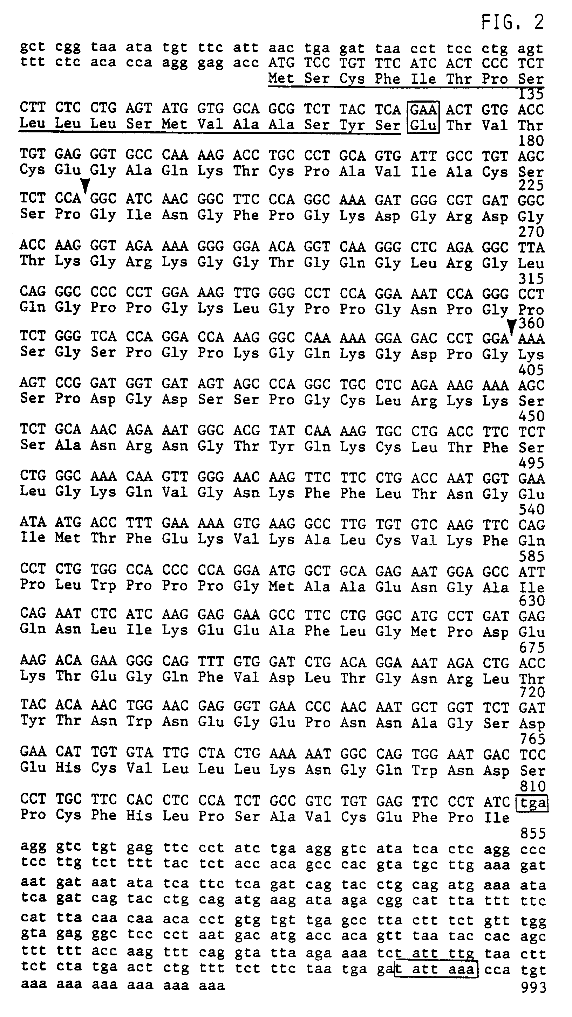

In one aspect, the invention features engineered nucleic acid fragments of at least about 60 contiguous bases of the nucleic acid encoding fragments of human mannose binding protein deposited in the ATCC as strain number 67483 which comprises at least about 60 bases from region 359-807 shown in Fig.2.

The nucleic acid is able to hybridize under hybridizing conditions to nucleic acid encoding human mannose-binding protein. By engineered nucleic acid is meant nucleic acid removed from its natural environment by recombinant DNA methodology, or synthetic nucleic acid, or cDNA. This nucleic acid may be a fragment of DNA or RNA, it may be present in a vector system,

The other aspects, the invention features vectors, and expression vectors or cells containing these vectors, each vector having the engineered nucleic acid, and the invention features peptides expressed from these vectors or cells. By peptide is meant a chain of two or more amino acids, including fragments of proteins and polypeptides. These peptides, and antibodies to these peptides, may be used as therapeutic or diagnostic agents.

In preferred embodiments, the nucleic acid encodes for a peptide having a greater than 75% homology to a fragment of at least thirty amino acids of human mannose-binding protein.

In other preferred embodiments the nucleic acid is cDNA; the hybridizing conditions are at 42°C in 5 x SSC, with washing at 68°C in 0.1 x SSC; and the nucleic acid encodes a carbohydrate binding region. Most preferably the region has at least about 60 bases from region 309-714, shown in Fig. 2; the nucleic acid is ligated to nucleic acid encoding the toxic part of a toxin molecule, most preferably the toxin molecule is chosen from AZT, ricin, or cholera toxin; the cell is a virus, bacterium, fungus, or eucaryotic cell; the virus is vaccinia, the bacterium is Escherichia coli, the fungus is yeast, and the eucaryotic cell is a cultured cell line.

The peptide encoded by the nucleic acid of the invention can be used in a method for treating animals infected with a bacterium, fungus, or virus. The method entails providing a peptide able to bind the mannose units on these organisms. The peptide is able to cause host defensive cells to be attracted to the organisms. The method further entails administering the peptide to the animal.

The peptide is a fragment of human mannose binding protein able to bind a carbohydrate; this peptide is able to disable the bacterium, fungus, or virus, and is a peptide as described above. Most preferably, the animal is human; the infection results in a bacteremia or local bacterial infection, parasitic infection, or fungal colonization, and the route of administration is either intravenous, intramuscular, oral, or local, i.e., in the form of a powder, or lotion; or the virus is HIV or a related virus, and the peptide lowers the rate of infection of eucaryotic cells by the virus; the protein or peptide is the mannose binding protein provided at 1-500 µg/ml final concentration in human serum or tissue.

In another aspect, the nucleic acid of the invention may be used in a method for diagnosing patients susceptible to infection by viruses, bacteria, parasites or fungi, the method features detecting the serum level of mannose-binding proteins in an animal, wherein this level reflects the susceptibility of the animal to an infection.

Preferably, the method features detecting reaction of an antibody to the above peptides with the serum, most preferably the detecting comprises an ELISA test.

Other features and advantages of the invention will be apparent from the following description of the preferred embodiments thereof.

### Description of the Preferred Embodiments

The drawings will first briefly be described.

### Drawings

Fig. 1 is a restriction endonuclease map of the MBP-human cDNA insert in pMBP.

Fig. 2 is a representation of the cDNA sequence and corresponding amino acid sequence of MBP human.

Fig. 3 is a representation of the genomic DNA, and corresponding amino acid sequences in all three reading frames of human MBP.

Fig. 4 is a diagram of a proposed model of MBP.

Fig. 5 is a comparison of the amino acid sequences of MBP-human with other lectins; invariant regions are shown on the top line, and galactose and mannose-specific regions on the lower lines.

### Human Mannose-binding protein (MBP-human)

MBP-human is a soluble lectin-like molecule which is synthesized in hepatocytes and released into the bloodstream. Generally, MBP-human is able to bind carbohydrates, such as mannose, at its carbohydrate binding domain. MBP-human can be isolated generally as described by Wild et al., *supra*, and Drickamer et al. *supra*, for example, by passage down a mannose-sepharose column.

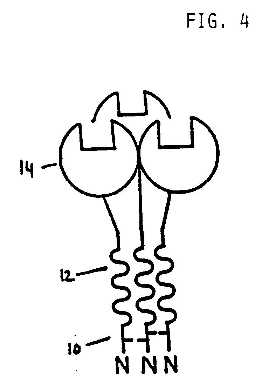

The general structure of MBP-human is shown in Fig. 4. The amino-terminal end 10 is cysteine rich, consistent with multimer formation by interchain disulfide bridges. Next to this is a collagen-like segment 12 having a repeated pattern of Gly-X-Y (Gly represents glycine; X and Y are other amino acids), similar to those of non-filbrillar collagen genes. Finally, there is a carboxy-terminal carbohydrate recognition domain 14. The mannose-binding domain is within the region.

Nucleic acid, for example, DNA, encoding MBP-human can be isolated by standard techniques. For example, oligonucleotide probes specific for the nucleic acid may be constructed and used to probe either genomic or cDNA libraries, as described by Drickamer et al., supra. Alternatively, gene fragments from related genes can be used as probes. Preferably, the probe is homologous to a region of the carbohydrate binding domain of MBP-human. The clones isolated by this technique contain engineered nucleic acid. Once isolated, the gene encoding MBP-human is useful for producing recombinant MBP-human protein, or peptide fragments thereof. In addition, the nucleic acid can be modified by standard techniques in order to express modified peptides.

Examples of cloning MBP-Human encoding nucleic acid are given below. These examples are not limiting to the invention and one skilled in the art will recognize that there are many equivalent means for accomplishing similar results.

### Example 1: cDNA clones

A human liver cDNA library was constructed in pKT218 by standard technique as described by Woods et al. 79 Proc. Natl.Acad. Sci. USA. 5661, 1982. This library was probed using a gel purified radiolabelled rat MBP-C cDNA sequence digested with XhoI and EcoRI as described by Drickamer et al., supra. This probe was used under non-stringent conditions to identify Potentially useful clones. The filters were prehybridized for 1 hour at 42°C in 0.75 M NaCl, 50mM sodium phosphate, pH7.4, 5mM EDTA, 5x Dehardts solution and 0.1% SDS (5 x SSC), and then hybridized overnight at 42°C. The filters were washed at 45°C in 2xSSC for 30 minutes and then in 1xSSC for 30 minutes. In addition a λHEPG2 gt10 cDNA library plated in E. coli C600 was screened, as described by Kwiatkowski et al. 323 Nature 455, 1986.

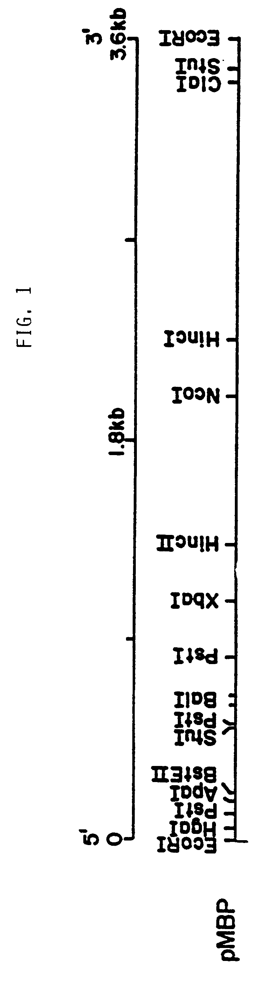

Five clones, including pMBP, were isolated and their sequences determined by the method of Sanger et al. (74 Proc. Natl. Acad. Sci. USA 5463, 1977) using M13, Mp18 cloning vectors (Messing et al. Proc. Nat. Acad. Sci. USA 74:3642, 1977). This sequence is shown in Fig. 2. The restriction map of pMBP is shown in Fig. 1, it has a 3.6kb EcoRI insert isolated from the above λ gt 10 library.

### Example 2: Genomic clone

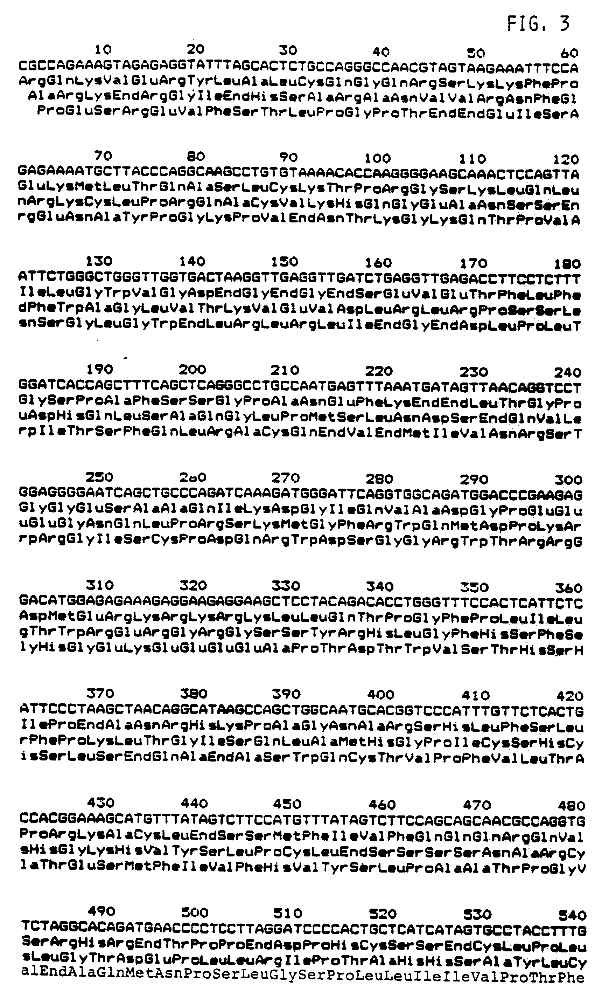

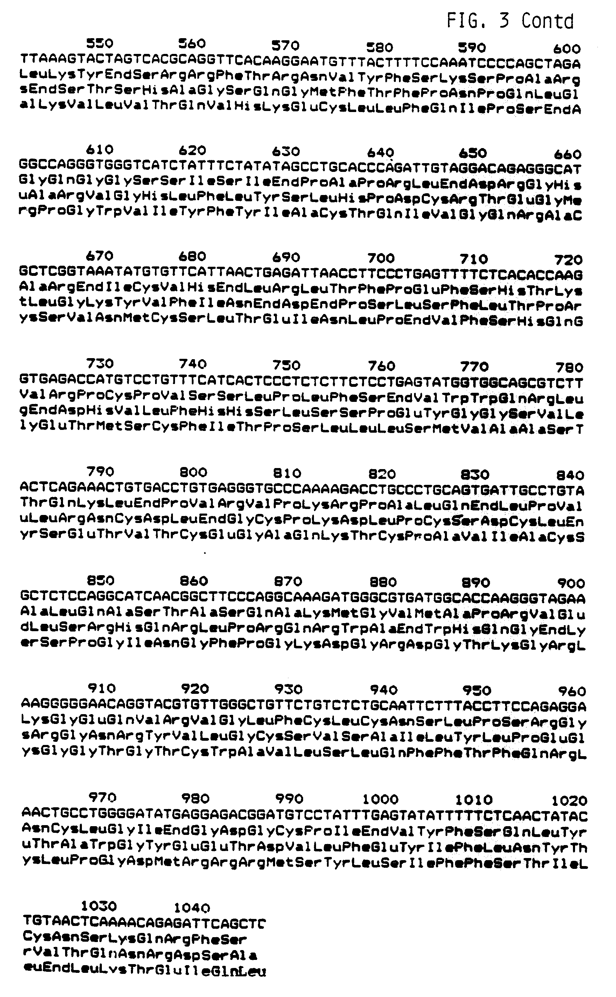

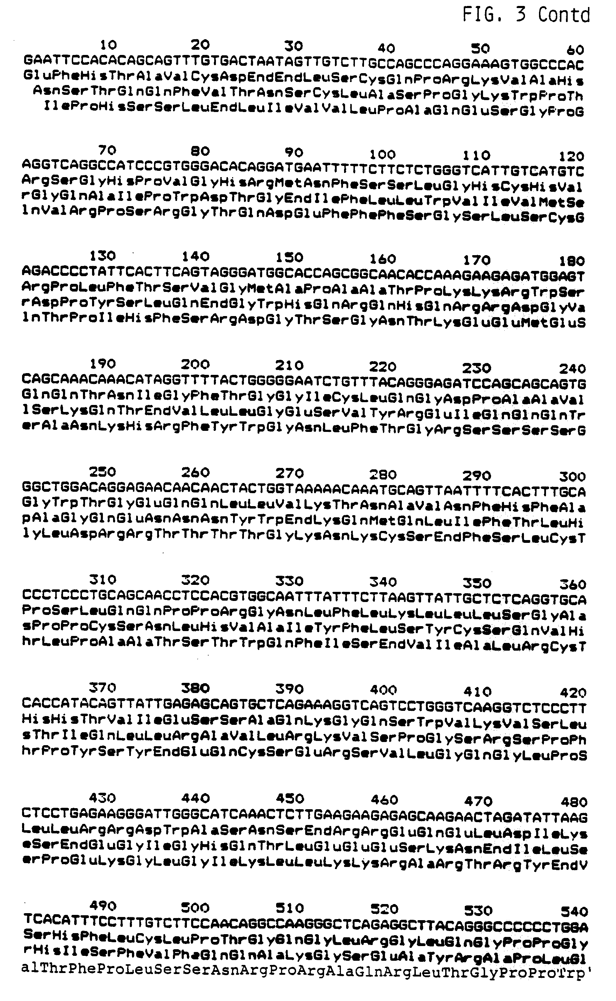

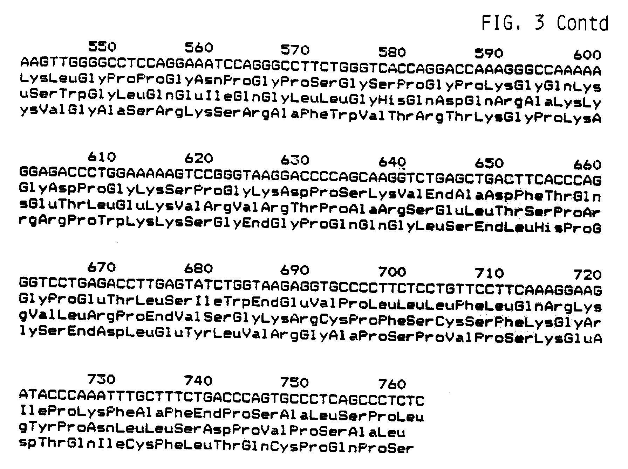

The 650 bp carboxy terminal Pst-1 fragment (Fig. 1) of a MBP-human cDNA clone was used as a probe for human genomic library. This library was contructed by standard techniques in EMBL 3A by inserting Mbol-digested genomic DNA into the BamHI site. Clones which hybridized under stringent conditions were isolated. Specifically, the hybridization was performed as described above, except the wash conditions were at 68°C in 0.1xSSC. The positively identified clones were plaque purified and their nucleic acid sequence determined as above. This sequence is presented in Fig. 3.

Other related genes can be isolated by this procedure. For example, the membrane receptor protein of macrophages is similar to MBP-human in that its DNA hybridizes under less stringent conditions (using the above hybridization buffer at 37°C) to MBP-human probes, and a peptide of similar size to MBP-human is immunoprecipitated with antisera to MBP-human.

Expression of MBP-human peptide fragments is by standard procedure. For example, the desired region of the MBP-human encoding DNA, preferably the cDNA, can be isolated from one of the above-described clones and inserted into any one of several standard expression vectors. A preferred region for expression is that encoding the carbohydrate binding domain, most preferably the mannose binding domain. This region is between nucleotide bases 359-807 in Fig. 2, including a 350bp Pst1-XbaI fragment. To identify the desired region more specifically the sequence is compared to that in related proteins such as human mannose receptor. Comparison to rat A and C MBPs reveals most homology between other mannose binding proteins at the region equivalent to the collagen region, at nucleotide bases 287-359 (Fig. 2). This region is not useful in the invention since it is not involved with mannose binding. Rather, the region from 359-807 in Fig. 2 is most useful.

In order to show that any particular region of MBP-human does bind mannose the cDNA encoding it can be engineered by standard procedures to produce clones containing just this region. The resulting cloned DNA is then inserted into an expression vector. The peptide produced by such a vector is then passed through a mannose-sepharose column to see whether it will bind to mannose. Alternatively, a radioimmunoassay can be performed to see if radiolabelled mannose will react with the expressed peptide. Those peptides which bind mannose are useful in this invention.

It is unlikely that a single short linear region of amino acids of the MBP-human peptide is involved in binding to mannose, rather two or more such regions will probably cooperate to form a three-dimensional peptide configuration which can interact with, and bind, mannose. Such regions can be identified by comparison to other mannose-binding proteins as described above, and the DNA fragment encoding all such regions cloned and expressed. Such a DNA fragment is likely to be at least 60-90 base pairs in length, encoding at least about 20-30 amino acids.

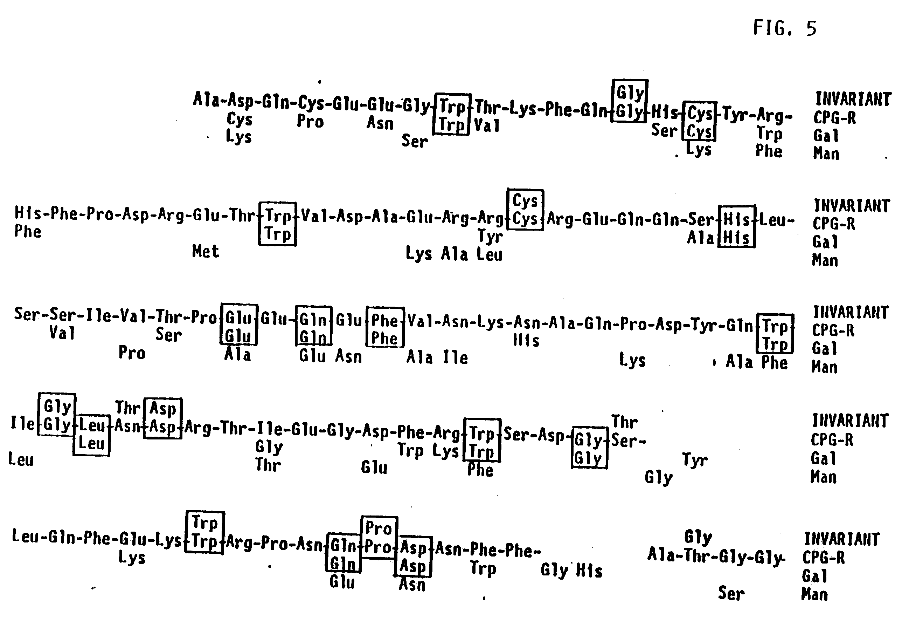

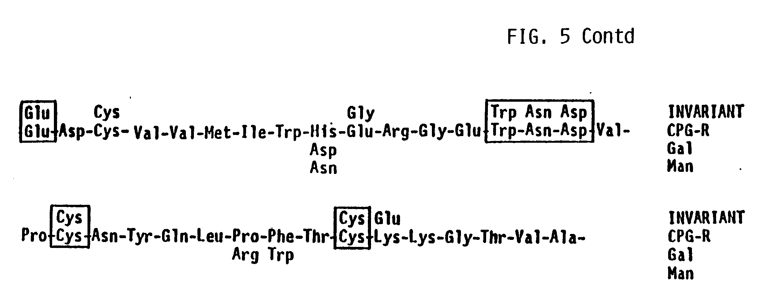

Referring to Fig. 5, such a comparison was performed by comparing other lectins, with mannose or other sugar binding specifities, to MBP-human. The lower line of the figure shows a concensus for mannose binding proteins, the amino acids on this line and in the upper line (showing invariant amino acids) are the most important for binding to mannose. These results were obtained by comparison of MBP-human to lectin proteins including the human and rat hepatic asialoglycoprotein receptors, the avian heptic receptor, the apoprotein of dog (Benson et al., Proc. Natl. Acad. Sci. USA 82:6379, 1985) and human surfactant (White et al., Nature 317:361, 1985); the NH₂ portion of a glactose specific lectin isolated from the hemolymph of S. periginia (Takahashi et al., J. Biol. Chem. 260:12228, 1985); a lectin isolated from the coelomic fluid of a sea urchin A. crassispina (Giga et al., J. Biol. Chem. 13: 6197, 1987); a chicken cartilage core proteoglycan protein (Shigaku et al., Proc. Natl. Acad. Sci. USA 83:5081, 1986) and the IgE Fc receptor (Ikuta et al., Proc. Natl. Acad. Sci. USA 84:819, 1987).

The above described mannose binding peptide, or the entire recombinant protein, is useful for specifically targeting cells expressing mannose on their surface, e.g., bacteria, fungi, and viruses. Thus, by linking this peptide to molecules able to kill or inhibit growth of such cells a hybrid peptide of great therapeutic use can be constructed. For example, the toxic part of ricin and cholera toxin, or chemicals such as AZT can be linked to this peptide. In order to do this, the nucleic acid encoding such toxins can be ligated to the mannose binding peptide-encoding nucleic acid and expressed as a single entity to form a hybrid peptide, for example, as described by Murphy U.S. Patent 4,675,382. Alternatively, the two peptides can be synthesized separately and linked chemically, for example, as described by Ross U.S. Patent 4,275,000,

Expression vectors suitable for peptide expression include all standard bacterial (e.g., pKK233-2, Amann et al. Gene in press, sold by Pharmacia, 800 Centennial Avenue, Piscataway, NJ 08854), yeast, and viral expression vectors, as well as eucaryotic vectors. Those skilled in the art will realize that such vectors generally are suitable for expressing the protein and the example below is not limiting to this invention.

The full length or partial cDNA MBP clone, with or without toxin peptide-encoding nucleic acid, can be ligated into the vector pSV2neo (Southern et al. J. Mol. Appl. Genet. 1:327 (1982) and Cloning Vectors, A Laboratory manual Ed. Pouwels et al. Elsevier Science Pub. NY, 52 Vanderbilt Avenue, NY, NY 10017, 1985) which contains an origin of replication from pBR322 and an ampicillin resistance gene. It also contains SV40 sequences to provide a transcriptional promoter and a polyadenylation sequence. The DNA is inserted between these two sequences. After ligation the recombinant vector is propagated in Escherichia coli and then introduced into Chinese hamster ovary cells using a standard calcium phosphate transfection protocol. The neo gene on this vector provides resistance to G418, which can be used to select for transformed cells.

Expression of mannose binding peptides by these vectors and organisms can be followed using a sepharose-mannose column. Expressed material is bound to the column, eluted with 50mM Tris/10mM EDTA, and run in 8% polyacrylamide gels (using Laemmli buffers, Nature 227:600, 1970) to observe the presence of peptides. Those clones which produce mannose-binding peptides, i.e., peptides which bind to such a column, are suitable in this invention.

Antibodies to such expressed peptides or to MBP-human itself can be produced by standard techniques. They may be monoclonal or polyclonal and are useful for identification of the peptides within animal serum or in clinical diagnostic tests.

### Use

Exposed mannose is a feature of the cell walls of many pathogens, whereas higher organisms, including humans and animals, tend to have processed membrane glycoproteins having complex sugars which mask internal mannose residues. These internal mannose residues are not recognized by MBPs. Recombinant mannose binding protein, or chimeric peptides containing the mannose binding domain, are useful therapeutic agents. These proteins or peptides specifically bind mannose-rich pathogens, including bacteria, fungi, yeasts, parasites, or the envelope glycoproteins of certain viruses, and thus direct removal of such pathogens from the animal.

For non-viral pathogens, efficacy of removal by host defense mechanisms may be increased by directing attachment of the mannose binding protein complex to the surface of phagocyte cells, thereby enhancing the clearance of the pathogens from the circulation, by causing the phagocytes to recognize these pathogens.

For viruses, which express mannose-rich glycoproteins, direct inactivation of the virus and viral infected cells is enhanced by attaching toxins, such as ricin, cholera, or diptheria or antimetabolite drugs, such as AZT, to the mannose binding domain of the mannose binding protein.

For example, the 350bp Pst1-XbaI fragment shown in Fig. 1, comprising the carboxy-terminal mannose binding domain of MBP-human can be expressed in an expression vector, and the peptide produced linked chemically to nucleotide analogues such as dideoxycytosine or AZT. As shown below, fluorescencely labelled such peptides do not bind to cells uninfected with HIV, the virus thought to cause Acquired Immunodeficiency Syndrome (AIDS), but do bind to infected cells. The resulting product should be particularly effective in specifically targeting drug-like molecules to HIV or HIV-infected cells.

### Example 3: HIV targeting

MBP-human was shown to be effective in vivo for preventing infection of H9 CD4⁺ cells with HIV. Purified HIV was incubated in the presence or absence of highly purified homogenous MBP-human (prepared as described by Summerfield et al., Biochimica et Biop. Acta 883:197, 1986; Wild et al., Biochem. J. 210:167, 1983; Townsend et al., Biochem. J. 194:209, 1981; and Kawasaki et al., J. Biochem. 94:937, 1983). The treated virus was then incubated with H9 CD4⁺ lymphocytes (which are primary targets for HIV infection) and 7 days later viral infectivity was measured by a) the appearance of HIV envelope glycoprotein (which was assayed on the cell surface by immunofluorescence using specific anti-envelope glycoprotein antisera) and b) the presence of reverse transcriptase activity (which is present only when the cell is infected with HIV). MBP-human completely inhibited viral entry into cells. This was shown by the absence of HIV envelope glycoprotein on the cell surface, and by indectectable reverse transcriptase activity. Control experiments showed that the inhibition by MBP-human was specific; these experiments involved completing MBP-human with mannose rich yeast mannan, and neo-glycoprotein mannose-BSA.

In experiments using fluorescently-labelled MBP-human to observe binding to infected or uninfected cells, the fluorescently labelled MBP-human was used to show that mannan and mannose-BSA inhibits the binding of MBP-human to virally infected cells, and that MBP-human does not bind uninfected H9 cells. Thus MBP-human is recognizing exposed mannose units on these cells.

Thus, MBP-human or the mannose binding domain thereof are suitable for identifying cells infected with HIV, or related viruses which express mannose rich envelope glycoproteins on their cell surface. The MBP-human, the mannose binding domain or chimeric molecules thereof can be used to target cytotoxic agents to directly and specifically kill infected cells. Further, these molecules can be used to prevent the spread of viral infection, and even the initial infection itself.

MBP-human and related peptides as described above may be administered by routine methods. For example, they can be injected directly into the blood stream of an animal, especially humans, to a level of between 1-500µg/ml serum (most preferably, 150µg/ml final concentration, and this dose repeated to maintain this level. They can be administered prophylaticaly or after infection. Similarly, the molecules may be administered orally, injected subcutaneously, or even applied in powder or lotion form, for example, to treat local infections, such as bacterial infection, or infection with Trichophyton rubrum, which causes athlete's foot.

Another use of these peptides is in the determination of an animal's susceptibility to infection by agents such as HIV. Here, the serum level of MBPS in the animal is measured using antibodies produced to MBP-human, or related peptides, in for example, an ELISA protocol. The level of MBPs in the serum can then be related to the susceptibility to infection of this animal to an agent, and this relationship used to estimate other animals' susceptibility. Thus, for example if a high level of MBP-human is linked to low susceptibility to infection by HIV, then a human having a low level of MBP-human is likely to be susceptible to HIV infection. Further, at the genomic level, such susceptibility may be related to defects in the nucleic acid. Such defects can be discovered using the cloned MBP-human genes, or fragments thereof, as probes. Polymorphisms linked to HIV susceptibility can be detected and used to predict susceptibility of other humans to infection.

### Deposits

The following deposit was made on August 4, 1987, with the American Type Culture Collection (ATCC), where the deposit was given the accession number ATCC 67483.

## Claims

1. A nucleic acid fragment of at least about 60 contiguous bases of the nucleic acid encoding human mannose binding protein deposited in the ATCC as strain number 67483 which comprises at least about 60 bases from region 359-807 shown in Fig. 2, and which fragment encodes a mannose binding peptide which is a fragment of said human mannose binding protein.

2. The fragment of claim 1 which is ligated to nucleic acid encoding the toxic part of a toxin molecule.

3. The fragment of claim 2 wherein said toxin molecule is chosen from AZT, ricin, or cholera toxin.

4. A cell comprising the nucleic acid fragment of any one of claims 1 to 3.

5. The cell of claim 4 wherein said cell is a bacterium, fungus, or eucaryotic cell.

6. The cell of claim 5 wherein said virus is vaccinia, said bacterium is Escherichia coli, said fungus is yeast, and said eucaryotic cell is a cultured cell line.

## Patentansprüche

1. Nukleinsäurefragment mit wenigstens etwa 60 aufeinanderfolgend Base der für menschliches mannosebindendes Protein kodierenden Nukleinsäure, welche in der ATCC als Stamm Nr. 67483 hinterlegt ist, wobei das Nukleinsäurefragment wenigstens etwa 60 Basen aus dem Bereich 359 - 807, dargestellt in Figur 2, umfaßt und ein mannosebindendes Peptid kodiert, welches ein Fragment des menschlichen mannosebindenden Proteins ist.

2. Fragment nach Anspruch 1, dadurch gekennzeichnet, daß es mit Nukleinsäure, die für den toxischen Bereich eines Toxinmoleküls kodiert, ligiert ist.

3. Fragment nach Anspruch 2, dadurch gekennzeichnet, daß das Toxinmolekül ausgewählt ist aus AZT, Ricin oder Choleratoxin.

4. Zelle, welche das Nukleinsäurefragment nach einem der Ansprüche 1 bis 3 enthält.

5. Zelle nach Anspruch 4, dadurch gekennzeichnet, daß die Zelle ein Bakterium, ein Pilz oder eine eukaryontische Zelle ist.

6. Zelle nach Anspruch 5, dadurch gekennzeichnet, daß das Virus Vaccinia, das Bakterium Eschericia coli, der Pilz Hefe und die eukaryontische Zelle eine kultivierte Zellinie ist.

## Revendications

1. Fragment d'acide nucléique d'au moins 60 bases contiguës environ de l'acide nucléique codant la protéine humaine liant le mannose déposée à l'ATCC sous le numéro de souche 67483 qui comprend au moins 60 bases environ de la région 359 à 807 représentée dans la figure 2, et lequel fragment code un peptide liant le mannose qui est un fragment de ladite protéine humaine liant le mannose.

2. Fragment selon la revendication 1 qui est lié à l'acide nucléique codant la partie toxique d'une molécule de toxine.

3. Fragment selon la revendication 2 dans lequel ladite molécule de toxine est choisie dans le groupe constitué par l'AZT, la ricine ou la toxine du choléra.

4. Cellule comprenant le fragment d'acide nucléique selon l'une quelconque des revendications 1 à 3.

5. Cellule selon la revendication 4 dans laquelle ladite cellule est une bactérie, un champignon ou une cellule eucaryotique.

6. Cellule selon la revendication 5 dans laquelle ledit virus est une vaccine, ladite bactérie est Escherichia coli, ledit champignon est une levure et ladite cellule eucaryotique est une lignée cellulaire cultivée.
